# EUROPEAN PATENT APPLICATION

(11) **EP 4 316 472 A1**
(43) Date of publication of application: **07.02.2024**
(21) Application number: 22781149.4
(22) Date of filing: 30.03.2022
(51) Int. Cl.: A61K 9/62, A61K 47/36

(54) **DISINTEGRABLE SEAMLESS CAPSULE THAT IS STABLE EVEN IN HIGH-TEMPERATURE AND HIGH-HUMIDITY ENVIRONMENT, AND METHOD FOR PRODUCING SAID SEAMLESS CAPSULE**

(30) Priority: 31.03.2021 JP 2021061495
(71) Applicant: Morishita Jintan Co., Ltd., Osaka-shi, Osaka 540-8566 (JP)
(72) Inventor: NAGAE, Kentarou, Hirakata-shi, Osaka 573-0128 (JP); NISHIKAWA, Takehiro, Hirakata-shi, Osaka 573-0128 (JP); NAKANO, Osami, Hirakata-shi, Osaka 573-0128 (JP); NAKATA, Yunosuke, Hirakata-shi, Osaka 573-0128 (JP); ISHII, Katsutoshi, Hirakata-shi, Osaka 573-0128 (JP)
(74) Representative: dompatent von Kreisler Selting Werner - Partnerschaft von Patent- und Rechtsanwälten mbB
(86) International application number: PCT/JP2022/016185
(87) International publication number: WO 2022/210939

(57) **Abstract**

The present invention provides a seamless capsule that does not soften even in a high-temperature and high-humidity environment, maintains hardness in a tropical zone, etc., and can perceive the breakage of the capsule provided by applying a force to the capsule. The present invention relates to a seamless capsule including a capsule content and a shell layer that coats the capsule content, and a method for producing the same. The shell layer comprises at least deacylated gellan gum and modified starch. The capsule content comprises an oily component.

## Description

### TECHNICAL FIELD

The present invention relates to a seamless capsule and a method for producing the same, and more particularly, to a seamless capsule stably disintegrable even in a high-temperature and high-humidity environment and a method for producing the same.

### BACKGROUND ART

Seamless capsules are used in many applications from the viewpoint of ease of particle size control, and simplicity of production, and the like. In particular, capsules enclosing useful bacteria, and capsules enclosing flavors such as menthol, and the like are commercially available. In recent years, the application thereof has been expanded, and not only a technique of increasing the strength of a capsule shell to make it difficult to break the capsules as in the conventional case, but also a technique of causing the capsules to stably exist even in a high-temperature and high-humidity environment such as a tropical zone is also required.

WO2005/077419A (Patent Literature 1) proposes that an enzymatically decomposed lecithin is added to a capsule formulation in order to prevent the adhesion of the capsule under high temperature and high humidity. This technique is a technique of preventing the adhesion of the capsule formulation under high temperature and high humidity. However, there is also a case of requiring a seamless capsule that can maintain the hardness of a capsule shell material even under a high temperature and high humidity environment, and perceive the breakage of the capsule provided by applying a force to the capsule. In particular, in a tropical region such as Bangkok in Thailand, which is hot and humid in a tropical monsoonal climate and is stuffy throughout the year, seamless capsules having such characteristics may be required.

### CITATIONS LIST

### PATENT LITERATURE

Patent Literature 1: WO2005/077419A

### SUMMARY OF INVENTION

### TECHNICAL PROBLEMS

The present invention provides a seamless capsule that does not soften even in a high-temperature and high-humidity environment, maintains hardness in a tropical zone, etc., and can perceive the breakage of the capsule provided by applying a force to the capsule.

### SOLUTIONS TO PROBLEMS

As a result of intensive studies to solve the above object, the present inventors have found that the above object is achieved by using a specific shell material for the capsule.

The present invention provides the following aspects.
[1] A seamless capsule including: a capsule content; and a shell layer that coats the capsule content. The shell layer comprises at least deacylated gellan gum and modified starch. The capsule content comprises an oily component.
[2] The seamless capsule according to [1], wherein the shell layer comprises the deacylated gellan gum in an amount of 70% by weight or more and 98.5% by weight or less based on a shell weight and the modified starch in an amount of 0.01% by weight or more and 30% by weight or less based on the shell weight.
[3] The seamless capsule according to [1] or [2], wherein the shell layer further comprises crystalline cellulose.
[4] The seamless capsule according to any one of [1] to [3], wherein the seamless capsule has a particle size of 0.5 to 8.0 mm and a shell ratio of 5% or more and 40% or less.
[5] The seamless capsule according to any one of [1] to [4], wherein the modified starch is an oxidized starch.
[6] The seamless capsule according to any one of [1] to [5], wherein the oily component in the capsule content comprises a flavor.
[7] The seamless capsule according to any one of [1] to [6], wherein the flavor is at least one selected from the group consisting of spearmint, menthol, peppermint, orange, yuzu, rose, lemon, grapefruit, kumquat, peach, apple, banana, pineapple, grape, mango, tropical fruit, blueberry, cranberry, lingonberry, huckleberry, raspberry, blackberry, loganberry, salmonberry, boysenberry, strawberry, bilberry, elderberry, and gooseberry.
[8] The seamless capsule according to any one of [1] to [7], wherein the seamless capsule has a two-layer structure including the capsule content and the shell layer, or a three-layer structure including an intermediate layer between the capsule content and the shell layer.
[9] The seamless capsule according to any one of [1] to [8], wherein a value of XY/xy is within a range of 0.76 or more and 1.15 or less, where X (N) is a load required to break the seamless capsule by pressing in a minimum particle size direction after the seamless capsule is stored for 60 minutes under a condition of a relative humidity of 90% RH at 60°C, and Y (mm) is a displacement at break, and similarly, x (N) is a load required to break the seamless capsule by pressing in a minimum particle size direction after the seamless capsule is stored for 120 minutes under a condition of a relative humidity of 60% RH at 25°C, and y (mm) is a displacement at break.
[10] The seamless capsule according to any one of [1] to [8], wherein a value of XY/xy is within a range of 0.76 or more and 1.32 or less, where X (N) is a load required to break the seamless capsule by pressing in a minimum particle size direction after the seamless capsule is stored for 60 minutes under a condition of a relative humidity of 90% RH at 60°C, and Y (mm) is a displacement at break, and similarly, x (N) is a load required to break the seamless capsule by pressing in a minimum particle size direction after the seamless capsule is stored for 120 minutes under a condition of a relative humidity of 60% RH at 25°C, and y (mm) is a displacement at break.
[11] A method for producing the seamless capsule according to [1] using a dropping method for simultaneously discharging liquids from a double nozzle in which an inner nozzle and an outer nozzle are concentrically present, and dropping into a coolant, the method comprising:
   discharging a capsule content liquid from the inner nozzle;
   discharging a shell layer liquid from the outer nozzle;
   controlling a temperature of the capsule content liquid to a set value ±2°C in a range of 5°C to 30°C, the capsule content liquid comprising an oily component;
   controlling a temperature of the shell layer liquid to a set value ±2°C in a range of 60°C to 90°C, the shell layer liquid comprising at least deacylated gellan gum and modified starch;
   controlling a temperature of the coolant to a set value ±2°C in a range of 10°C to 30°C, the coolant comprising oil; and
   controlling a difference between the temperature of the shell layer liquid and the temperature of the coolant in a range of 30°C or higher and 80°C or lower.
[12] A method for producing the seamless capsule according to [1] using a dropping method for simultaneously discharging liquid from a triple nozzle in which an inner nozzle, an intermediate nozzle, and an outer nozzle are concentrically present, and dropping into a coolant, the method comprising:
   discharging a capsule content liquid from the inner nozzle;
   discharging an intermediate layer liquid from the intermediate nozzle;
   discharging a shell layer liquid from the outer nozzle;
   controlling a temperature of the capsule content liquid to a set value ±2°C in a range of 5°C to 30°C, the capsule content liquid comprising an oily component;
   controlling a temperature of the intermediate layer liquid to a set value ±2°C in a range of 40 to 85°C;
   controlling a temperature of the shell layer liquid to a set value ±2°C in a range of 60°C to 90°C, the shell layer liquid comprising at least deacylated gellan gum and modified starch;
   controlling a temperature of the coolant to a set value ±2°C in a range of 10°C to 30°C, the coolant comprising oil; and
   controlling a difference between the temperature of the shell layer liquid and the temperature of the coolant in a range of 30°C or higher and 80°C or lower.
[13] The method for producing the seamless capsule according to [11] or [12], wherein the modified starch is an oxidized starch.
[14] The method for producing the seamless capsule according to any one of [11] to [13], wherein the shell layer further comprises crystalline cellulose.
[15] The method for producing the seamless capsule according to any one of [11] to [14], wherein the oily component in the capsule content liquid comprises a flavor.
[16] The method for producing the seamless capsule according to any one of [11] to [15], wherein the flavor is at least one selected from the group consisting of spearmint, menthol, peppermint, orange, yuzu, rose, lemon, grapefruit, kumquat, peach, apple, banana, pineapple, grape, mango, tropical fruit, blueberry, cranberry, lingonberry, huckleberry, raspberry, blackberry, loganberry, salmonberry, boysenberry, strawberry, bilberry, elderberry, and gooseberry.

### ADVANTAGEOUS EFFECTS OF INVENTION

In the present invention, the deacylated gellan gum and the modified starch are used for the capsule shell layer of the seamless capsule, and therefore the seamless capsule does not soften in a high-temperature and high-humidity environment, maintains hardness, and can maintain sound emission upon breaking, even if the shell ratio of the seamless capsule is low.

### BRIEF DESCRIPTION OF DRAWINGS

FIG. 1 is a schematic diagram of a seamless capsule having a three-layer structure according to an embodiment of a disintegrable capsule.
FIG. 2 is a schematic cross-sectional view of a nozzle portion of a producing apparatus suitable for producing a seamless capsule having a three-layer structure according to a dropping method using a three-layer nozzle.

### DESCRIPTION OF EMBODIMENTS

The present invention provides a seamless capsule including: a capsule content; and a shell layer that coats the capsule content, wherein: the shell layer comprises deacylated gellan gum and modified starch; and the capsule content comprises an oily component. The seamless capsule may have a two-layer structure including the capsule content and the shell layer, or a three-layer structure including an intermediate layer between the capsule content and the shell layer. In the case of the seamless capsule having a three-layer structure, the intermediate layer may be partially or entirely turbid or mixed with the capsule content so that the intermediate layer cannot be clearly recognized. Hereinafter, the seamless capsule having a three-layer structure will be described with reference to the drawings.

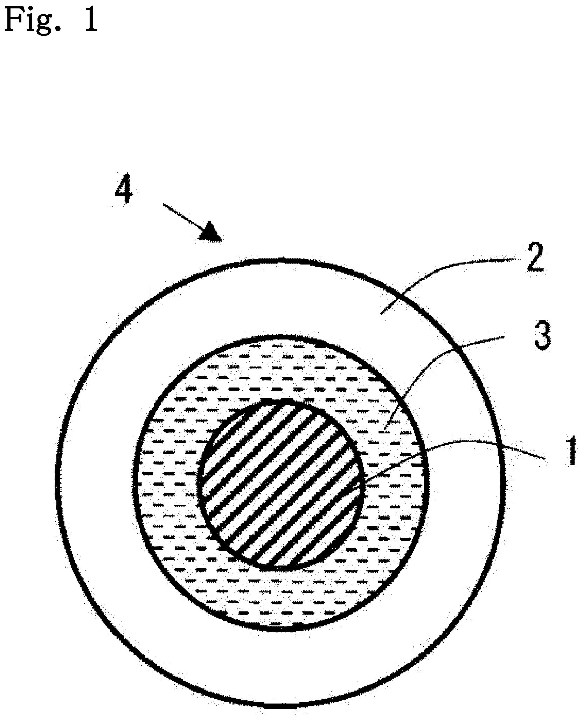

FIG. 1 shows a schematic diagram of an embodiment of a disintegrable capsule. As shown in FIG. 1, a seamless capsule 4 is a seamless capsule including a capsule content 1 and a shell layer 2, and also including an intermediate layer 3 as necessary. The capsule content 1 is enclosed in the shell layer 2. The content 1 of the capsule comprises an oily component such as a flavor, and the shell layer 2 comprises deacylated gellan gum and modified starch. The seamless capsule 4 is disintegrable, and when a pressure is applied to the seamless capsule with fingers, the shell layer 2 is ruptured, and the capsule content 1 is released to the outside of the capsule. Specifically, when the shell layer 2 is broken, the flavor or the like which is the oily component of the capsule content 1 is released, and a user can enjoy the scent of the flavor. In the present specification, "disintegrable" refers to that the capsule shell can be disintegrated by an external pressure to release the content.

### <Capsule content>

The capsule content can take any of an aspect containing water as a main component and an oily component, an aspect containing an oily component as a main component, and an aspect composed only of an oily component. The phrase "containing an oily component as a main component" specifically means that the oily component accounts for, for example, 50% by weight or more, preferably 60% by weight or more, more preferably 70% by weight or more, and still more preferably 80% by weight or more, with respect to the total weight of the capsule content. The phrase "containing water as a main component and an oily component" specifically means that the water component accounts for, for example, 80% by weight or less, preferably 70% by weight or less, and more preferably 60% by weight or less, with respect to the total weight of the capsule content.

Examples of the oily component include, but not particularly limited to, a flavor, a grain oil, a fruit oil, and a lipophilic solvent. The oily component preferably comprises a flavor, and more preferably comprises a flavor and a lipophilic solvent. The oily component may be prepared as an oil/water/oil type emulsion using known materials. The oily component may be used singly or in combination of two or more kinds thereof.

The flavor is not particularly limited as long as it is an oily component, and either a natural flavor or a synthetic flavor may be used. The flavor may be used singly or in combination of two or more kinds thereof. Examples of the natural flavor include, but not particularly limited to, oils such as rose, jasmine, citrus aurantium neroli, chamomile, ylang ylang, geranium, eucalyptus, tea tree, putigrain, unshiu mikan, orange, lemon, lime, bergamot, pepper, juniper, vanilla, sandalwood, pine, cypress, cinnamon, frankincense, myrrh, vetiver, spike nard, orris root, lavender, lemongrass, basil, rosemary, mints (for example, spearmint, menthol, peppermint, and the like), and berries (for example, blueberry, cranberry, lingonberry, huckleberry, raspberry, blackberry, loganberry, salmonberry, boysenberry, strawberry, bilberry, elderberry, and quince berry and the like). The synthetic flavor is not particularly limited as long as it is conventionally used for the purpose of imparting aromatic odor and flavor. Examples thereof include esters, alcohols, aldehydes, ketones, phenols, ethers, lactones, hydrocarbons, nitrogen-containing compounds, sulfur-containing compounds, acids, and mixtures thereof.

Examples of the esters used as the flavor of the oily component include, but not particularly limited to, propyl formate, butyl formate, amyl formate, octyl formate, linalyl formate, citronellyl formate, geranyl formate, neryl formate, terpinyl formate, ethyl acetate, isopropyl acetate, isoamyl acetate, hexyl acetate, cis-3-hexenyl acetate, trans-2-hexenyl acetate, octyl acetate, nonyl acetate, decyl acetate, dodecyl acetate, dimethyl undecadienyl acetate, styryl acetate, ocimenyl acetate, myrcenyl acetate, dihydromyrcenyl acetate, linalyl acetate, citronellyl acetate, geranyl acetate, neryl acetate, tetrahydromugol acetate, labanduryl acetate, nerolidol acetate, dihydrocumyl acetate, terpinyl acetate, citryl acetate, nopyru acetate, dihydroterpinyl acetate, 2,4-dimethyl-3-cyclohexenylmethyl acetate, miraldyl acetate, beticol acetate, decenyl propionate, linalyl propionate, geranyl propionate, neryl propionate, terpinyl propionate, tricyclodecenyl propionate, styralyl propionate, anisyl propionate, octyl butyrate, neryl butyrate, cinnamyl butyrate, isopropyl isobutyrate, octyl isobutyrate, linalyl isobutyrate, neryl isobutyrate, linalyl isovalerate, terpinyl isovalerate, phenylethyl isovalerate, 2-methyl pentyl 2-methylvalerate, methyl 3-hydroxyhexanoate, ethyl 3-hydroxyhexanoate, methyl octanoate, octyl octanoate, linalyl octanoate, methyl nonanoate, methyl undecylenate, linalyl benzoate, methyl cinnamate, isoprenyl angelate, methyl geranate, triethyl citrate, ethyl acetoacetate, ethyl 2-hexylacetoacetate, ethyl benzylacetoacetate, allyl 2-ethylbutyrate, ethyl 3-hydroxybutyrate, ethyl nonanoate, ethyl decanoate, ethyl 2,4-decadienate, propyl 2,4-decadienate, methyl and linalyl anthranilate, and ethyl N-methylanthranilate.

Examples of the alcohols used as the flavor of the oily component include, but not particularly limited to, 3-heptanol, 1-nonanol, n-undecanol, 2-undecanol, n-dodecanol, prenol, 10-undecene-1-ol, dihydrolinalool, tetrahydromugol, myrcenol, dihydromyrcenol, tetrahydromyrcenol, ocimenol, terpineol, hotrienol, 3-thujanol, benzyl alcohol, β-phenylethyl alcohol, α-phenylethyl alcohol, 3-methyl-1-pentanol, 1-heptanol, 2-heptanol, 3-octanol, 1-nonanol, 2-nonanol, 2,6-dimethylheptanol, 1-decanol, trans-2-hexanol, cis-4-hexanol, methyltrimethylcyclopentenyl butenol, cytolonerol, dihydromyrcenol, rosinol, geraniol, nerol, linalool, tetrahydrolinalool, dimethyloctanol, hydroxycitronellol, isopulegol, menthol (for example, 1-menthol), terpineol, dihydroterpineol, carbeol, dihydrocarbeol, perilla alcohol, 4-thujanol, myrtenol, α-fenchyl alcohol, farnesol, nerolidol, cedrenol, anise alcohol, hydrotropic alcohol, 3-phenylpropyl alcohol, cinnamic alcohol, and amyl cinnamic alcohol.

Examples of the aldehydes used as the flavor of the oily component include, but not particularly limited to, acetaldehyde, n-hexanal, n-heptanal, n-octanal, n-nonanal, 2-methyloctanal, 3,5,5-trimethylhexanal, decanal, undecanal, 2-methyldecanal, dodecanal, tridecanal, tetradecanal, trans-2-hexenal, trans-4-decenal, cis-4-decenal, trans-2-decenal, 10-undecenal, trans-2-undecenal, trans-2-dodecenal, 3-dodecenal, trans-2-tridecenal, 2,4-hexadienal, 2,4-decadienal, 2,4-dodecadienal, 5,9-dimethyl-4,8-decadienal, citral, dimethyloctanal, α-methylenecitronellal, citronelloxyacetaldehyde, myrtenal, neral, α- or β-sinensal, myrac aldehyde, phenylacetaldehyde, octanal dimethyl acetal, nonanal dimethyl acetal, decanal dimethyl acetal, decanal diethyl acetal, 2-methylundecanal dimethyl acetal, citral dimethyl acetal, citral diethyl acetal, citral propylene glycol acetal, n-valeraldehyde, isovaleraldehyde, 2-methylbutanal, 2-pentenal, trans-2-heptenal, trans-2-nonenal, 2,6-dimethyl-5-peptenal, 2,4-undecadienal, trimethyldecadienal, citronellal, hydroxycitronellal, safranal, vernaldehyde, benzaldehyde, p-isopropylphenylacetaldehyde, p-methyl hydrotropic aldehyde, phenylpropionaldehyde, 2-methyl-3-(4-methylphenyl)propanal, cyclamenaldehyde, cinnamic aldehyde, salicylaldehyde, anisealdehyde, p-methylphenoxyacetaldehyde, acetaldehyde diethyl acetal, citronellyl methyl acetal, acetaldehyde 2-phenyl-2,4-pentanediol acetal, 2-hexenal diethyl acetal, cis-3-hexenal-diethyl acetal, heptanal diethyl acetal, 2-hexyl-5-methyl-1,3-dioxolane, citronellal cyclomonoglycol acetal, hydroxycitronellal dimethyl acetal, and phenylacetaldehyde dimethyl acetal.

Examples of the ketones used as the flavor of the oily component include, but not particularly limited to, 2-pentanone, 3-hexanone, 2-heptanone, 3-heptanone, 4-heptanone, 2-octanone, 3-octanone, 2-nonanone, 2-undecanone, methylheptenone, dimethyloctenone, geranylacetone, farnesylacetone, 2,3,5-trimethyl-4-cyclohexenyl-1-methyl ketone, nerone, nootkatone, dihydronootkatone, acetophenone, 4,7-dihydro-2-isopentyl-2-methyl-1,3-dioxepin, 2-pentanone, 3-hexanone, 2-heptanone, 2,3-hexadione, 3-nonanone, ethyl isoamyl ketone, diacetyl, amyl cyclopentenone, 2-cyclopentyl cyclopentanone, hexylcyclopentanone, heptylcyclopentanone, cis-jasmone, dihydrojasmone, trimethylpentylcyclopentanone, 2-(2-(4-methyl)-3-cyclohexene-1-yl)propylcyclopentanone, damascone, α-dynascone, trimethylcyclohexenylbutenone, α-ionone, β-ionone, methylionone, allylionone, plicatone, cashmeran, l-carvone, menthone, camphor, p-methylacetophenone, p-methoxyacetophenone, benzylideneacetone, raspberry ketone, methyl naphthyl ketone, benzophenone, furfural acetone, homofuronol, maltol, ethyl maltol, and ethyl acetoacetate ethylene glycol ketal.

Examples of the phenols used as the flavor of the oily component include, but not particularly limited to, thymol, carvacrol, β-naphthol isobutyl ether, anethole, β-naphthol methyl ether, β-naphthol ethyl ether, creosol, veratrole, hydroquinone dimethyl ether, 2,6-dimethoxyphenol, 4-ethylguaiacol, eugenol, isoeugenol, ethyl isoeugenol, and tert-butylhydroquinone dimethyl ether.

Examples of the ethers used as the flavor of the oily component include, but not particularly limited to, decyl vinyl ether, α-terpinyl methyl ether, isoproxene, 2,2-dimethyl-5-(1-methyl-1-propenyl)-tetrahydrofuran, rosefuran, 1,4-cineol, nerol oxide, 2,2,6-trimethyl-6-vinyl tetrahydropyran, methylhexyl ether, ocimene epoxide, limonene oxide, rhubofix, caryophyllene oxide, linalool oxide, 5-isopropenyl-2-methyl-2-vinyltetrahydrofuran, nerol oxide, and rose oxide.

Examples of the lactones used as the flavor of the oily component include, but not particularly limited to, γ-undecalactone, δ-dodecalactone, γ-hexalactone, γ-nonalactone, γ-decalactone, γ-dodecalactone, j asminlactone, methyl-γ-decalactone, 7-decenolactone, j asmolactone, propylidenephthalide, δ-hexalactone, δ-2-decenolactone, ε-dodecalactone, dihydrocoumarin, and coumarin.

Examples of the hydrocarbons used as the flavor of the oily component include, but not particularly limited to, ocimene, limonene, α-phellandrene, terpinene, 3-carene, bisabolene, valencene, alloocimene, myrcene, farnesene, α-pinene, β-pinene, camphene, terpinolene, p-cymene, cedrene, β-caryophyllene, and cadinene.

Examples of the nitrogen-containing compound or sulfur-containing compound used as the flavor of the oily component include, but not particularly limited to, methyl anthranilate, ethyl anthranilate, methyl N-methylanthranilate, methyl N-2'-methylpentylideneanthranilate, ligantral, dodecanenitrile, 2-tridecenenitrile, geranylnitrile, citronellylnitrile, 3,7-dimethyl-2,6-nonadienenitrile, indole, 5-methyl-3-heptanone oxime, limonenethiol, 1-p-menthene-8-thiol, butyl anthranilate, cis-3-hexenyl anthranilate, phenylethyl anthranilate, cinnamyl anthranilate, dimethyl sulfide, and 8-mercaptomenthone.

In the capsule content of the present invention, an excipient, a stabilizer, a surfactant, an auxiliary agent, or a foaming agent or the like may be further appropriately blended. The amounts of such additives are not particularly limited, but do not inhibit the function of the seamless capsule of the present invention.

### intermediate layer>

In the seamless capsule of the present invention, an intermediate layer is formed outside the capsule content. The intermediate layer is at least one layer, and may be composed of a plurality of layers. In the case of the plurality of layers, the layers may be used in the same formulation or in different formulations, but the intermediate layer is usually one layer. Hereinafter, for simplicity, the intermediate layer will be described as one layer. In the intermediate layer, examples of the oily composition that can be used for preparing the capsule content include olive oil, jojoba oil, corn oil, rapeseed oil, lard, beef tallow, whale oil, beeswax, castor oil, soybean oil, rice oil, rice germ oil, coconut oil, palm oil, cacao oil, avocado oil, macadamia nut oil, squalene, mink oil, turtle oil, corn oil, medium-chain fatty acid triglycerides (MCT), long-chain fatty acid triglycerides (LCT), hydrocarbons having 8 to 30 carbon atoms, beeswax, carnauba wax, rice wax, lanolin, liquid paraffin, petrolatum, fatty acids having 4 to 30 carbon atoms, esters of fatty acids having 4 to 30 carbon atoms and sucrose, esters of fatty acids having 4 to 30 carbon atoms and glycerol, aliphatic alcohols having 4 to 30 carbon atoms, esters of fatty acids having 4 to 30 carbon atoms and aliphatic alcohols having 4 to 30 carbon atoms, and silicone oils. Only one of these may be used, or two or more thereof may be mixed and used. Among these oily compositions, a liquid oil and fat having a viscosity of 200 mPa·s or less in a temperature range of - 30°C to 60°C is more preferable.

In the intermediate layer, lecithin or sucrose acetate isobutyrate (SAIB), and silicon dioxide may be blended alone or in combination in order to adjust interfacial tension, a viscosity, and a specific gravity. The amounts of such additives are not particularly limited, but do not inhibit the function of the seamless capsule of the present invention.

### <Shell layer>

The intermediate layer is further coated with a shell layer. The shell layer comprises deacylated gellan gum and modified starch. Gellan gum (also referred to as jellan gum) is a water-soluble polysaccharide produced by gram-negative bacterium Pseudomonas elodea, and is a linear heteropolysaccharide composed of repeating units of four sugars of glucose, glucuronic acid, glucose, and rhamnose. There are two types of gellan gum: deacylated gellan gum and native gellan gum, depending on the presence or absence of an acetyl group and a glyceryl group present in 1-3 bonded glucose. In the present invention, deacylated gellan gum is used. As the deacylated gellan gum, a commercially available product is used, and examples thereof include KELCOGEL (trademark) F and GELZAN (trademark) CM (both manufactured by CPKelco).

The deacylated gellan gum is contained in the shell layer in an amount of 70% by weight or more and 98.5% by weight or less based on a shell weight. The amount of the deacylated gellan gum is preferably 70% by weight or more and 97% by weight or less. When the amount of the deacylated gellan gum is less than 70% by weight, the hardness of the capsule shell is insufficient, and therefore no sound is emitted upon breaking the capsule shell. When the amount of the deacylated gellan gum is more than 98% by weight, the capsule shell tends to be too hard.

The shell layer also includes modified starch (also called "processed starch"). The starch includes unmodified starch and modified starch, and examples of the unmodified starch include any one of corn starch, waxy corn starch, tapioca starch, potato starch, rice starch, wheat starch, and sago starch, or a combination thereof. The modified starch is obtained by modifying (or processing) these unmodified starches, and is obtained by performing any one of oxidation, acid treatment, gelatinization, acetylation, etherification, phosphate crosslinking, and adipic acid crosslinking, or a combination thereof.

The acid treatment of the unmodified starch has effects such as a decrease in a viscosity and improvement in transparency and viscosity stability. The gelatinization has a cold water solubilizing effect. The acetylation has effects such as a decrease in a gelatinization temperature, improvement in transparency and water retainability, and aging resistance. The oxidation of the unmodified starch has effects such as improvement in working efficiency upon cooking due to a decrease in the viscosity of the starch, and improvement in transparency. When the starch is dissolved in water or the like, the solid content of a sheet can be increased by the oxidation of the unmodified starch without increasing the viscosity of the paste liquid, and therefore the oxidation of the unmodified starch has also an effect of improving viscosity stability. The etherification of the starch has effects such as a decrease in the adhesion temperature of a soft capsule due to a decrease in a gelatinization temperature, improvement in transparency and water retainability, aging resistance, salt resistance, and shear resistance, and also has an advantage that the sheet can be easily formed. Examples of the etherification include hydroxypropylation. The phosphoric acid crosslinking of the starch has an effect of preventing viscosity reduction and gel disintegration due to acid, alkali, and mechanical shear, and making the starch film water-resistant. The acetylation has effects such as a decrease in the adhesion temperature of a soft capsule due to a decrease in a gelatinization temperature and improvement in transparency. The modified starch used in the present invention is preferably an oxidized starch.

In the present invention, the shell layer comprises the modified starch in an amount of 0.01% by weight or more and less than 30% by weight based on a shell weight. The modified starch is preferably contained in the shell layer in an amount of 0.02% by weight or more and 25% by weight or less. When the amount of the modified starch is less than 0.01% by weight, the hardness of the shell layer is insufficient, and when the amount of the modified starch is more than 30% by weight, the shell layer tends to be brittle.

The shell layer of the seamless capsule of the present invention may comprise a plasticizer as necessary in a dry state, and examples of the plasticizer include glycerin and sorbitol. The blending amount of the plasticizer is 1 to 50% by mass, preferably 5 to 40% by mass, and more preferably 15 to 30% by mass, based on the total weight of the dried shell. When the blending amount of the plasticizer is less than 1% by mass, the shell cannot withstand vacuum drying, or cannot maintain sufficient flexibility in a dry state, to result in cracking of the shell. When the blending amount of the plasticizer is more than 50% by mass, the shell softens, and therefore the adhesion and melting of the shell occur at a high temperature.

The shell layer of the seamless capsule of the present invention may comprise various additives commonly used in this field, such as crystalline cellulose, a cellulose derivative, a flavor, a sweetener, a colorant, and a preservative such as paraben, in addition to the above composition, as necessary. When such additives are used, the total content of all the additives is, for example, 0.01% by weight to 10% by weight, and preferably 0.1% by weight to 5% by weight, based on the total solid content weight in the shell layer composition of the capsule.

The crystalline cellulose is also referred to as microcrystalline cellulose, and is obtained by partially depolymerizing α-cellulose obtained from a fibrous plant with an acid and purifying the depolymerized α-cellulose. The crystalline cellulose has glucose units linked by β (1 → 4) glycosidic bonds, and is a compound that is insoluble in water, dilute acids, and most organic solvents but has hygroscopicity. AVICEL PH series, CELEX101, CEOLUS, MCOSER, and VIVACELL series are commercially available from companies. The cellulose derivative is a compound in which a hydroxyl group of the cellulose is substituted with an alkyl ether (-O-M) group or a fatty acid ester group (-C(=O)-M) (M: alkyl), and has a property of being dissolved in water or the like.

The diameter (D) (mm) of the seamless capsule of the present invention is not particularly limited, and can be set according to a situation in which the seamless capsule is used. In an embodiment, the diameter (D) of the capsule is 0.5 to 8.0 mm, and preferably 1.0 to 7.0 mm.

The shell ratio (%) of the shell layer of the seamless capsule of the present invention after drying is given by {(thickness of shell) × 2/D × 100, and is preferably 5% or more and 40% or less, and desirably 10% to 35%. When the shell ratio is less than 5%, the shell strength is low, and when the shell ratio is more than 40%, the amount of the content is reduced, and the disintegrability of the shell layer is also deteriorated.

The diameter and breaking strength of the capsule can be adjusted within the scope of the present invention by adjusting the composition and amount of the shell of the seamless capsule, adjusting the composition and amount of the capsule content liquid, and making adjustments in capsule production steps (for example, a cooling step and a drying step and the like in the dropping-in-liquid method described later).

For example, in the case of a seamless capsule having a diameter of about 3.5 mm, the total weight of the seamless capsule of the present invention is preferably 17 to 30 mg, more preferably 18 to 27 mg, and still more preferably 19 to 25 mg.

In a preferable embodiment of the seamless capsule of the present invention, the capsule shell is ruptured by applying an external pressure to the seamless capsule. In a preferable embodiment, the capsule pops and makes rupturing sound upon rupturing. The shape of the capsule is not particularly limited, and may be, for example, a sphere shape or a football shape. The sphere shape is preferable, and a perfect sphere shape is more preferable. In the case of the perfect sphere shape, more specifically, the short diameter/long diameter ratio of the capsule is not particularly limited, and is preferably 0.90 to 1.00, and more preferably 0.95 to 1.00.

### <Breaking performance of seamless capsule>

As described above, the seamless capsule of the present invention has a specific breaking performance, specifically, a performance in which the capsule shell is ruptured by applying an external pressure, and preferably the seamless capsule pops and makes rupturing sound upon rupturing. Therefore, the seamless capsule of the present invention has the following breaking characteristics.

When X (N) is a load required to break the seamless capsule by pressing in a minimum particle size direction after the seamless capsule of the present invention is stored for 60 minutes under a condition of a relative humidity of 90% RH at 60°C, and similarly, x (N) is a load required to break the seamless capsule by pressing in a minimum particle size direction after the seamless capsule of the present invention is stored for 120 minutes under a condition of a relative humidity of 60% RH at 25°C, a value of X/x is not particularly limited, but is preferably within a range of 0.6 or more and 1.3 or less, and more preferably within a range of 0.7 or more and 1.2 or less.

When Y (mm) is a displacement at break required to break the seamless capsule by pressing in a minimum particle size direction after the seamless capsule of the present invention is stored for 60 minutes under a condition of a relative humidity of 90% RH at 60°C, and similarly, y (mm) is a displacement at break required to break the seamless capsule by pressing in a minimum particle size direction after the seamless capsule of the present invention is stored for 120 minutes under a condition of a relative humidity of 60% RH at 25°C, a value of Y/y is preferably within a range of 0.9 or more and 2.1 or less, and more preferably within a range of 1.0 or more and 2.0 or less.

Specifically, when the seamless capsule of the present invention comprises a content comprising an oily component as a main component, a value of XY/xy (a value obtained by dividing a product of X and Y by a product of x and y) is preferably within a range of 0.76 or more and 1.15 or less, where X (N) is a load required to break the seamless capsule by pressing in a minimum particle size direction after the seamless capsule is stored for 60 minutes under a condition of a relative humidity of 90% RH at 60°C, and Y (mm) is a displacement at break, and similarly, x (N) is a load required to break the seamless capsule by pressing in a minimum particle size direction after the seamless capsule is stored for 120 minutes under a condition of a relative humidity of 60% RH at 25°C, and y (mm) is a displacement at break. When such numerical ranges are satisfied, the seamless capsule is broken by an external pressure, and in some cases, popping rupturing sound is made. If the value of XY/xy is less than 0.76, the capsule has a disadvantage that when the value is less than 0.76, the strength of the capsule cannot be maintained under a high-temperature and high-humidity condition, and the capsule is apt to be broken. If the value is more than 1.15, the capsule has a disadvantage that the capsule is too soft under a similar condition, and is hardly broken. Descriptions in parentheses after x, X, y, and Y mean units, N is Newton, and mm is a millimeter. The "minimum particle size direction" is the direction of the short diameter since the direction of the short diameter is the most stable in consideration of the case where the seamless capsule has a football shape although the seamless capsule has a shape close to a substantially true sphere shape in terms of producing method.

Similarly, in the case of the capsule comprising a content comprising an aqueous component as a main component, if the value of XY/xy is less than 0.76, the capsule has a disadvantage that the strength of the capsule cannot be maintained under a high-temperature and high-humidity condition, and the capsule is apt to be broken. If the value is more than 1.32, the capsule has a disadvantage that the capsule is too soft under a similar condition, and is hardly broken.

In the present specification, the load at break and the displacement at break are measured by continuously applying a load to the seamless capsule particle in a vertical direction until the seamless capsule particle disintegrates. The maximum load at the moment when the capsule is broken is the breaking strength (N) of the capsule. The breaking strength (N) is measured using, for example, a rheometer (commercially available from Sun Scientific Co., Ltd.).

In the present specification, the particle size of the capsule can be evaluated by measuring with a digital caliper and taking an average value of the measured values. In the present specification, the shell thickness of the capsule can be evaluated by cutting the capsule in half, measuring with a digital microscope, and taking an average value of the measured values.

### <Method for producing seamless capsule>

The seamless capsule of the present invention is produced by a producing method referred to as so-called dropping method. The dropping method is a producing method utilizing a phenomenon in which a droplet is dropped from a nozzle into a fluid, to cause surface tension to form the sphere shape of the droplet. As described above, the seamless capsule of the present invention may have a two-layer structure including the capsule content and the shell layer, or may have a three-layer structure having an intermediate layer between the capsule content and the shell layer. Therefore, a producing method in the case of having the two-layer structure and a producing method in the case of having the three-layer structure will be described.

### (Method for producing seamless capsule having two-layer structure)

In a method for producing a seamless capsule using a dropping method for simultaneously discharging liquids from a double nozzle in which an inner nozzle and an outer nozzle are concentrically present, followed by dropping the liquids into a coolant, the seamless capsule is produced by: discharging a capsule content liquid from the inner nozzle; discharging a shell layer liquid from the outer nozzle; controlling a temperature of the capsule content liquid to a set value ±2°C (preferably ±1°C) in a range of 5°C to 30°C (preferably 10°C to 30°C), the capsule content liquid containing an oily component; controlling a temperature of the shell layer liquid to a set value ±2°C (preferably ±1°C) in a range of 60°C to 90°C, the shell layer liquid containing at least deacylated gellan gum and modified starch; controlling a temperature of the coolant to a set value ±2°C (preferably ±1°C) in a range of 10°C to 30°C (preferably 12°C to 30°C), the coolant containing oil; and controlling a difference between the temperature of the shell layer liquid and the temperature of the coolant in a range of 30°C or higher and 80°C or lower (preferably 35°C or higher and 73°C or lower).

### (Method for producing seamless capsule having three-layer structure)

In a method for producing a seamless capsule using a dropping method for simultaneously discharging liquids from a triple nozzle in which an inner nozzle, an intermediate nozzle, and an outer nozzle are concentrically present, followed by dropping them into a coolant, the seamless capsule is produced by: discharging a capsule content liquid from the inner nozzle; discharging an intermediate layer liquid from the intermediate nozzle; discharging a shell layer liquid from the outer nozzle; controlling a temperature of the capsule content liquid to a set value ±2°C (preferably ±1°C) in a range of 5°C to 30°C (preferably 10°C to 30°C), the capsule content liquid containing an oily component; controlling a temperature of the intermediate layer liquid to a set value ±2°C (preferably ±1°C) in a range of 40°C to 85°C (preferably 45 to 85°C); controlling a temperature of the shell layer liquid to a set value ±2°C (preferably ±1°C) in a range of 60°C to 90°C (preferably 65°C to 85°C), the shell layer liquid containing at least deacylated gellan gum and modified starch; controlling a temperature of the coolant to a set value ±2°C in a range of 10°C to 30°C (preferably 12°C to 30°C), the coolant containing oil; and controlling a difference between the temperature of the shell layer liquid and the temperature of the coolant in a range of 30°C or higher and 80°C or lower (preferably 35°C or higher and 73°C or lower).

The above temperature conditions can be appropriately selected by those skilled in the art according to the degree of quality required for the seamless capsule.

In producing the seamless capsule of the present invention, it is particularly important to satisfy all of the above conditions, and for example, those skilled in the art can easily perform the above temperature control by combining PID control with feedforward control, but the present invention is not limited to these control methods.

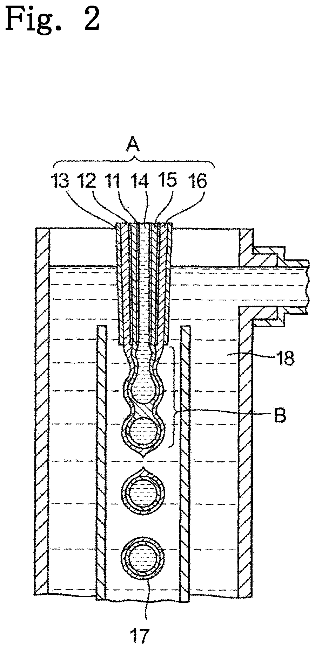

FIG. 2 is a schematic cross-sectional view of a nozzle portion of a producing apparatus suitable for producing a seamless capsule having a three-layer structure according to a dropping method using a three-layer nozzle.

FIG. 2 illustrates a state where a seamless capsule jet B discharged from a nozzle cross section A is broken and shaped in a coolant 18 to form each seamless capsule 17. In the nozzle cross section A, an inner nozzle 11, an intermediate nozzle 12, and an outer nozzle 13 are concentrically present. A capsule content liquid 14 is discharged from the inner nozzle 11. An intermediate layer liquid 15 is discharged from the intermediate nozzle 12 (specifically, between the intermediate nozzle 12 and the inner nozzle 11). A shell layer liquid 16 is discharged from the outer nozzle 13 (specifically, between the outer nozzle 13 and the intermediate nozzle 12). The three kinds of liquids are simultaneously discharged to form the seamless capsule jet B.

The seamless capsule obtained as described above is dried by air at 5°C to 30°C for 2 to 12 hours. When it is necessary to further lower the moisture content of the seamless capsule, the capsule may be further subjected to vacuum drying or vacuum-freeze drying after air drying. In the vacuum drying, a degree of vacuum is kept at 0.002 to 0.5 MPa or less, and in the vacuum-freeze drying, freezing is performed at -20°C or lower and drying is performed. A time required for the vacuum drying or the vacuum-freeze drying is not particularly limited, and is generally 5 to 60 hours, and preferably 24 to 48 hours. When the time is 5 hours or less, drying is insufficient, and water present in the capsule adversely affects the content.

### EXAMPLES

Next, the present invention will be more specifically described with reference to Examples, but the present invention should not be understood to be limited to these Examples. In Examples, parts and % and the like are based on weight or mass unless otherwise indicated.

### Example 1

Deacylated gellan gum, calcium chloride, oxidized starch, and blue No. 1 of parts by weight described in the section of a shell layer in the following Table 1, and 32333 parts by weight of water were mixed, dissolved at 95°C, left standing, and defoamed to prepare a capsule shell liquid.

The capsule shell liquid, an intermediate layer made of a mixture of 857 parts by weight of sucrose acetate isobutyrate and 143 parts by weight of a medium-chain fatty acid triglyceride, as well as a capsule content made of a mixture of 200 parts by weight of l-menthol and 800 parts by weight of a medium-chain fatty acid triglyceride were charged into a seamless capsule production apparatus. Each solution was discharged into a flowing medium-chain fatty acid triglyceride (about 30°C) with the capsule content set at a temperature of 10°C, and the capsule shell liquid and the intermediate layer set at a temperature of 60°C using a concentric triple nozzle to produce capsules. Then, the capsules were dried (25°C, humidity: 50% RH or less) to obtain seamless capsules having a particle size of 3.5 mm. In Table 1, the compositions and liquid temperatures of the intermediate layers and capsule content, and the liquid temperature of the cooling liquid were also described.

The diameters D (mm) and shell thicknesses T (µm) of the obtained dry seamless capsules were measured, and the shell ratios were calculated. The results were shown in Table 2. The diameter D (mm) of the dry seamless capsule was measured by a caliper, and the shell thickness T (µm) was measured by cutting the capsule in half, measuring 5 points per sphere with a digital microscope (digital microscope vhd-5000 manufactured by Keyence Corporation), and taking an average value thereof.

A load x (N) and a displacement at break y (mm) required for breaking each of the obtained dry seamless capsules immediately after the dry seamless capsule was subjected to an environment at 25°C and a humidity of 60% RH for 2 hours and a load X (N) and a displacement at break Y (mm) required for breaking the seamless capsule immediately after the seamless capsule was subjected to an environment at 60°C and a humidity of 90% RH for 2 hours were obtained by pressing a circular plunger having a diameter of 10 mm using a comprehensive physical property measuring apparatus (Rheometer manufactured by Sun Scientific Co., Ltd.) and evaluating the seamless capsules at a table speed of 20 mm/min. Average breaking strengths x(N) = Σₙ₌₁²⁰xₙ/20, X(N) = Σₙ₌₁²⁰Xₙ/20, average values of displacements y(mm) = Σₙ₌₁²⁰yₙ/20, Y(mm) = Σₙ₌₁²⁰Yₙ/20, XY/xy, X/x, and Y/y measured for the 20 capsules of the same specification are listed in Table 2.

The obtained seamless capsule was held with fingers, and subjected to an environment of 60°C and a humidity of 90% RH for 2 hours. Immediately afterwards, a force was applied with the fingers. As a result, the seamless capsule easily popped, and was broken. The breaking sound and feeling of the obtained seamless capsule were enjoyed. The scent of menthol was also enjoyed. In Table 2, the state of breakage as "sound" was described according to the following evaluation criteria.
∘: High pitch and large amplitude of sound
△: Low pitch and small amplitude of sound
×: No sound

### Examples 2 to 7

Seamless capsules were prepared in the same manner as in Example 1 except that the compositions of shell layers, intermediate layers, and capsule contents were the compositions shown in Table 1. For the obtained seamless capsules, D (mm), T (µm), shell ratios, x (N), y (mm), X (N), and Y (mm) were measured in the same manner as in Example 1 to determine XY/xy, X/x, and Y/y. The results are described in Table 2. Breaking sound and feeling were also measured in the same manner as in Example 1, and the evaluations of "sound" were described in Table 2.

### Example 8

There were used the shell liquid of Example 1, and a capsule content liquid being a mixture made of 122 parts by weight of lemon oil, 532 parts by weight of medium-chain fatty acid triglyceride, and 264 parts by weight of sucrose acetate isobutyrate. The solutions were charged into a concentric double nozzle, with the shell liquid heating to 82°C and the content liquid set at 25°C to produce capsules. Then, the capsules were dried (25°C, humidity: 50% RH or less) to obtain seamless capsules. For the obtained seamless capsules, D (mm), T (µm), a shell ratio, x (N), y (mm), X (N), and Y (mm) were measured in the same manner as in Example 1 to determine XY/xy, X/x, and Y/y. The results are described in Table 2. The obtained seamless capsule was held immediately after being subjected to an environment of 60°C and a humidity of 90% RH for 2 hours, and a force was applied with a finger. As a result, only dull sound was generated, and in Table 2, the "sound" was evaluated as △.

**[Table 1]**

| | | Examples | | | | | | | |
|---|---|---|---|---|---|---|---|---|---|
| | | 1 | 2 | 3 | 4 | 5 | 6 | 7 | 8 |
| Shell layer | Deacylated gellan gum | 970 | 900 | 800 | 700 | 600 | 600 | 600 | 970 |
| | Calcium chloride dihydrate | 20 | 20 | 20 | 20 | 20 | 20 | 20 | 20 |
| | Oxidized starch | 8 | 78 | 178 | 278 | 378 | 378 | 378 | 8 |
| | Crystalline cellulose | | | | | | | | |
| | Blue No. 1 | 2 | 2 | 2 | 2 | 2 | 2 | 2 | 2 |
| | Purified water | 32333 | 32333 | 27571 | 19000 | 32333 | 14385 | 14385 | 32333 |
| | Temperature of shell layer liquid (°C) | 95 | 90 | 85 | 80 | 80 | 80 | 80 | 95 |
| Intermediate layer | Sucrose acetate isobutyrate | 857 | 857 | 857 | 857 | 857 | 857 | 857 | |
| | Medium-chain fatty acid triglyceride | 143 | 143 | 143 | 143 | 143 | 143 | 143 | |
| | Temperature of intermediate layer liquid (°C) | 60 | 50 | 50 | 50 | 60 | 60 | 50 | |
| Capsule content | Medium-chain fatty acid triglyceride | 800 | 800 | 800 | 800 | 800 | 800 | 800 | 532 |
| | L-menthol | 200 | 200 | 200 | 200 | 200 | 200 | 200 | |
| | Lemon oil | | | | | | | | 122 |
| | Carboxymethyl cellulose | | | | | | | | |
| | Sucrose acetate isobutyrate | | | | | | | | 264 |
| | Temperature of content | 10 | 15 | 20 | 10 | 15 | 15 | 15 | 35 |
| Coolant | Temperature of coolant (°C) | 30 | 30 | 25 | 30 | 20 | 20 | 30 | 15 |

**[Table 2]**

| | Examples | | | | | | | |
|---|---|---|---|---|---|---|---|---|
| | 1 | 2 | 3 | 4 | 5 | 6 | 7 | 8 |
| D (mm) | 3.5 | 3.5 | 3.5 | 3.5 | 3.5 | 3.5 | 6.0 | 3.6 |
| T (µm) | 37 | 37 | 37 | 37 | 37 | 37 | 38 | 60 |
| Shell ratio (%) | 8.00 | 8.00 | 8.00 | 8.00 | 8.00 | 8.00 | 5.00 | 13.00 |
| x (N) | 20.70 | 15.20 | 14.20 | 13.20 | 14.40 | 7.90 | 21.50 | 12.50 |
| y (mm) | 1.40 | 1.00 | 1.10 | 1.00 | 1.20 | 0.70 | 1.50 | 0.80 |
| X (N) | 17.00 | 11.70 | 10.10 | 10.30 | 12.70 | 10.50 | 18.50 | 9.10 |
| Y (mm) | 1.50 | 1.10 | 1.20 | 1.10 | 1.10 | 1.20 | 1.60 | 1.30 |
| XY/xy | 0.88 | 0.85 | 0.78 | 0.86 | 0.81 | 2.28 | 0.92 | 1.18 |
| X/x | 0.82 | 0.77 | 0.71 | 0.78 | 0.88 | 1.33 | 0.86 | 0.73 |
| Y/y | 1.07 | 1.10 | 1.09 | 1.10 | 0.92 | 1.71 | 1.07 | 1.63 |
| Sound | ○ | ○ | ○ | ○ | ○ | Δ | ○ | Δ |

### Example 9

There were used a capsule content liquid prepared by adding 4 parts by weight of carboxymethyl cellulose to 745 parts by weight of purified water, dissolving the mixture, then adding 216 parts by weight of glycerin, 10 parts by weight of propyl parahydroxybenzoate, and 25 parts by weight of lemon flavor, and sufficiently suspending the mixture; a liquid, being to an intermediate layer, prepared by dissolving 450 parts by weight of beeswax, 320 parts by weight of a hardened oil and fat, 200 parts by weight of sucrose acetate isobutyrate, and 30 parts by weight of soybean lecithin at 80°C; and a shell liquid described in Table 3. Using a concentric triple nozzle, the shell liquid was heated to 82°C, and the intermediate layer and the content liquid were respectively set at 60°C and 10°C to produce capsules. Then, the capsules were dried (25°C, humidity: 50% RH or less) to obtain seamless capsules. For the obtained seamless capsule, D (mm), T (µm), a shell ratio, x (N), y (mm), X (N), and Y (mm) were measured in the same manner as in Example 1 to determine XY/xy, X/x, and Y/y. The results are shown in Table 4. The obtained seamless capsule was held immediately after being subjected to an environment of 60°C and a humidity of 90% RH for 2 hours, and a force was applied with a finger. Breaking sound and feeling were also measured in the same manner as in Example 1, and the evaluations of "sound" were described in Table 4.

### Examples 10 to 16

Deacylated gellan gum, calcium chloride, oxidized starch, beeswax, and blue No. 1 of parts by weight described in the section of a shell layer in Table 3, and purified water were mixed, dissolved at 95°C, left standing, and defoamed to prepare a capsule shell liquid. There were further used a capsule content liquid and a mixture liquid, being to an intermediate layer, described in Table 3 and prepared in the same manner as in Example 9 while maintaining the temperature of each discharge liquid described in Table 3 using a concentric triple nozzle to produce capsules. Then, the capsules were dried (25°C, humidity: 50% RH or less) to obtain a seamless capsule. For the obtained seamless capsule, D (mm), T (µm), a shell ratio, x (N), y (mm), X (N), and Y (mm) were measured in the same manner as in Example 1 to determine XY/xy, X/x, and Y/y. The results are shown in Table 4. The obtained seamless capsule was held immediately after being subjected to an environment of 60°C and a humidity of 90% RH for 2 hours, and a force was applied with a finger. Breaking sound and feeling were also measured in the same manner as in Example 1, and the evaluations of "sound" were described in Table 4.

**[Table 3]**

| | | Examples | | | | | | | |
|---|---|---|---|---|---|---|---|---|---|
| | | 9 | 10 | 11 | 12 | 13 | 14 | 15 | 16 |
| Shell layer | Deacylated gellan gum | 960 | 890 | 790 | 690 | 590 | 590 | 770 | 970 |
| | Calcium chloride dihydrate | 20 | 20 | 20 | 20 | 20 | 20 | 20 | 20 |
| | Oxidized starch | 8 | 78 | 178 | 278 | 378 | 378 | 8 | 8 |
| | Blue No. 1 | 2 | 2 | 2 | 2 | 2 | 2 | 2 | 2 |
| | Crystalline cellulose | 10 | 10 | 10 | 10 | 10 | 10 | | |
| | Beeswax | | | | | | | 200 | |
| | Purified water | 32333 | 32333 | 27571 | 19000 | 14385 | 14385 | 32333 | 32333 |
| | Temperature of shell layer liquid (°C) | 95 | 90 | 85 | 80 | 80 | 80 | 80 | 95 |
| Intermediate layer | Beeswax | 450 | 450 | 450 | 450 | 450 | 450 | 300 | 450 |
| | Soy lecithin | 30 | 30 | 30 | 30 | 30 | 30 | 30 | 30 |
| | Sucrose acetate isobutyrate | 200 | 200 | 200 | 200 | 200 | 200 | 200 | 200 |
| | Hardened oil | 320 | 320 | 320 | 320 | 320 | 320 | 470 | 320 |
| | Temperature of intermediate layer liquid (°C) | 60 | 50 | 50 | 50 | 60 | 50 | 60 | 50 |
| Capsule content | Purified water | 745 | 745 | 745 | 745 | 745 | 745 | 928 | 670 |
| | Glycerin | 216 | 216 | 216 | 216 | 216 | 216 | 13 | 216 |
| | Lemon flavor | 25 | 25 | 25 | 25 | 25 | 25 | 25 | 100 |
| | Propyl paraoxybenzoate | 10 | 10 | 10 | 10 | 10 | 10 | 10 | 10 |
| | Carboxymethyl cellulose | 4 | 4 | 4 | 4 | 4 | 4 | 9 | 4 |
| | Temperature of content (°C) | 10 | 15 | 20 | 10 | 15 | 15 | 15 | 35 |
| Coolant | Temperature of coolant (°C) | 30 | 30 | 25 | 30 | 20 | 15 | 20 | 15 |

**[Table 4]**

| | Examples | | | | | | | |
|---|---|---|---|---|---|---|---|---|
| | 9 | 10 | 11 | 12 | 13 | 14 | 15 | 16 |
| D (mm) | 3.6 | 3.6 | 3.6 | 3.6 | 3.6 | 4.2 | 4.9 | 3.5 |
| T (µm) | 40 | 40 | 45 | 40 | 60 | 48 | 60 | 83 |
| Shell ratio (%) | 9.00 | 9.00 | 10.00 | 9.00 | 13.00 | 9.00 | 9.00 | 17.00 |
| x (N) | 14.30 | 14.90 | 14.20 | 14.90 | 13.60 | 18.00 | 23.20 | 14.90 |
| y (mm) | 1.00 | 1.00 | 1.10 | 1.00 | 1.10 | 1.10 | 1.50 | 0.90 |
| X (N) | 13.60 | 13.40 | 10.10 | 13.40 | 6.00 | 16.20 | 9.10 | 13.30 |
| Y (mm) | 1.10 | 1.20 | 1.20 | 1.20 | 1.30 | 1.50 | 1.40 | 1.50 |
| XY/xy | 1.05 | 1.08 | 0.78 | 1.08 | 0.52 | 1.23 | 0.37 | 1.49 |
| X/x | 0.95 | 0.90 | 0.71 | 0.90 | 0.44 | 0.90 | 0.39 | 0.89 |
| Y/y | 1.10 | 1.20 | 1.09 | 1.20 | 1.18 | 1.36 | 0.93 | 1.67 |
| Sound | ○ | ○ | ○ | ○ | △ | ○ | × | × |

### REFERENCE SIGNS LIST

- 1: Capsule content
- 2: Intermediate layer
- 3: Shell layer
- 4: Seamless capsule
- A: Nozzle cross section
- B: Seamless capsule jet
- 11: Inner nozzle
- 12: Intermediate nozzle
- 13: Outer nozzle
- 14: Capsule core liquid
- 15: Intermediate layer liquid
- 16: Outermost layer liquid
- 17: Seamless capsule having three-layer structure
- 18: Coolant

## Claims

1. A seamless capsule comprising:
a capsule content; and
a shell layer that coats the capsule content,
wherein
the shell layer comprises at least deacylated gellan gum and modified starch, and
the capsule content comprises an oily component.

2. The seamless capsule according to claim 1, wherein the shell layer comprises the deacylated gellan gum in an amount of 70% by weight or more and 98.5% by weight or less based on a shell weight and the modified starch in an amount of 0.01% by weight or more and 30% by weight or less based on the shell weight.

3. The seamless capsule according to claim 1 or 2, wherein the shell layer further comprises crystalline cellulose.

4. The seamless capsule according to any one of claims 1 to 3, wherein the seamless capsule has a particle size of 0.5 to 8.0 mm and a shell ratio of 5% or more and 40% or less.

5. The seamless capsule according to any one of claims 1 to 4, wherein the modified starch is an oxidized starch.

6. The seamless capsule according to any one of claims 1 to 5, wherein the oily component in the capsule content comprises a flavor.

7. The seamless capsule according to any one of claims 1 to 6, wherein the flavor is at least one selected from the group consisting of spearmint, menthol, peppermint, orange, yuzu, rose, lemon, grapefruit, kumquat, peach, apple, banana, pineapple, grape, mango, tropical fruit, blueberry, cranberry, lingonberry, huckleberry, raspberry, blackberry, loganberry, salmonberry, boysenberry, strawberry, bilberry, elderberry, and gooseberry.

8. The seamless capsule according to any one of claims 1 to 7, wherein the seamless capsule has a two-layer structure including the capsule content and the shell layer, or a three-layer structure including an intermediate layer between the capsule content and the shell layer.

9. The seamless capsule according to any one of claims 1 to 8, wherein a value of XY/xy is within a range of 0.76 or more and 1.15 or less, where X (N) is a load required to break the seamless capsule by pressing in a minimum particle size direction after the seamless capsule is stored for 60 minutes under a condition of a relative humidity of 90% RH at 60°C, and Y (mm) is a displacement at break, and similarly, x (N) is a load required to break the seamless capsule by pressing in a minimum particle size direction after the seamless capsule is stored for 120 minutes under a condition of a relative humidity of 60% RH at 25°C, and y (mm) is a displacement at break.

10. The seamless capsule according to any one of claims 1 to 8, wherein a value of XY/xy is within a range of 0.76 or more and 1.32 or less, where X (N) is a load required to break the seamless capsule by pressing in a minimum particle size direction after the seamless capsule is stored for 60 minutes under a condition of a relative humidity of 90% RH at 60°C, and Y (mm) is a displacement at break, and similarly, x (N) is a load required to break the seamless capsule by pressing in a minimum particle size direction after the seamless capsule is stored for 120 minutes under a condition of a relative humidity of 60% RH at 25°C, and y (mm) is a displacement at break.

11. A method for producing the seamless capsule according to claim 1 using a dropping method for simultaneously discharging liquids from a double nozzle in which an inner nozzle and an outer nozzle are concentrically present, and dropping into a coolant, the method comprising:
discharging a capsule content liquid from the inner nozzle;
discharging a shell layer liquid from the outer nozzle;
controlling a temperature of the capsule content liquid to a set value ±2°C in a range of 5°C to 30°C, the capsule content liquid comprising an oily component;
controlling a temperature of the shell layer liquid to a set value ±2°C in a range of 60°C to 90°C, the shell layer liquid comprising at least deacylated gellan gum and modified starch;
controlling a temperature of the coolant to a set value ±2°C in a range of 10°C to 30°C, the coolant comprising oil; and
controlling a difference between the temperature of the shell layer liquid and the temperature of the coolant in a range of 30°C or higher and 80°C or lower.

12. A method for producing the seamless capsule according to claim 1 using a dropping method for simultaneously discharging liquid from a triple nozzle in which an inner nozzle, an intermediate nozzle, and an outer nozzle are concentrically present, and dropping into a coolant, the method comprising:
discharging a capsule content liquid from the inner nozzle;
discharging an intermediate layer liquid from the intermediate nozzle;
discharging a shell layer liquid from the outer nozzle;
controlling a temperature of the capsule content liquid to a set value ±2°C in a range of 5°C to 30°C, the capsule content liquid comprising an oily component;
controlling a temperature of the intermediate layer liquid to a set value ±2°C in a range of 40 to 85°C;
controlling a temperature of the shell layer liquid to a set value ±2°C in a range of 60°C to 90°C, the shell layer liquid comprising at least deacylated gellan gum and modified starch;
controlling a temperature of the coolant to a set value ±2°C in a range of 10°C to 30°C, the coolant comprising oil; and
controlling a difference between the temperature of the shell layer liquid and the temperature of the coolant in a range of 30°C or higher and 80°C or lower.

13. The method for producing the seamless capsule according to claim 11 or 12, wherein the modified starch is an oxidized starch.

14. The method for producing the seamless capsule according to any one of claims 11 to 13, wherein the shell layer further comprises crystalline cellulose.

15. The method for producing the seamless capsule according to any one of claims 11 to 14, wherein the oily component in the capsule content liquid comprises a flavor.

16. The method for producing the seamless capsule according to any one of claims 11 to 15, wherein the flavor is at least one selected from the group consisting of spearmint, menthol, peppermint, orange, yuzu, rose, lemon, grapefruit, kumquat, peach, apple, banana, pineapple, grape, mango, tropical fruit, blueberry, cranberry, lingonberry, huckleberry, raspberry, blackberry, loganberry, salmonberry, boysenberry, strawberry, bilberry, elderberry, and gooseberry.
